## Europäisches Patentamt

## European Patent Office

## Office européen des brevets

(19)

(11) Veröffentlichungsnummer : **0 025 963**
**B1**

(12) **EUROPÄISCHE PATENTSCHRIFT**

(45) Veröffentlichungstag der Patentschrift :
**22.06.83**

(51) Int. Cl.³ : **B 01 J 23/96, C 07 D301/10**

(21) Anmeldenummer : **80105513.8**

(22) Anmeldetag : **13.09.80**

(54) Verfahren zur Verbesserung der Wirksamkeit von Silber-Trägerkatalysatoren.

(30) Priorität : **21.09.79 DE 2938245**

(43) Veröffentlichungstag der Anmeldung :
**01.04.81 Patentblatt 81/13**

(45) Bekanntmachung des Hinweises auf die Patenterteilung : **22.06.83 Patentblatt 83/25**

(84) Benannte Vertragsstaaten :
**BE DE FR GB IT NL SE**

(56) Entgegenhaltungen :
DE A 2 062 190
DE A 2 751 767
FR A 2 368 298
GB A 1 512 625

(73) Patentinhaber : **HOECHST AKTIENGESELLSCHAFT**
**Postfach 80 03 20**
**D-6230 Frankfurt/Main 80 (DE)**

(72) Erfinder : **Alfranseder, Josef**
**Hauptstrasse 31**
**D-8261 Markt1/Inn; (DE)**
Erfinder : **Mayer, Sigmund**
**Hochfeilnstrasse 20**
**D-8261 Burgkirchen/Alz; (DE)**
Erfinder : **Rebsdat, Siegfried, Dr.**
**Trenbeckstrasse 24**
**D-8261 Burg, Post Neuötting (DE)**
Erfinder : **Riedl, Josef, Dr.**
**Kantstrasse 53**
**D-8261 Burgkirchen/Alz; (DE)**
Erfinder : **Schaffelhofer, Iwo, Dr.**
**Ketteler Strasse 2**
**D-8263 Burghausen/Salzach (DE)**

# 0 025 963

## Verfahren zur Verbesserung der Wirksamkeit von Silber-Trägerkatalysatoren

Die Erfindung betrifft ein Verfahren zur Verbesserung der Wirksamkeit von Silber-Trägerkatalysatoren für die Direktoxidation von Ethylen zu Ethylenoxid mit molekularem Sauerstoff oder Luft, wobei der für die Direktoxidation gebrauchte Katalysator mit einer inerten Flüssigkeit gewaschen wird und hierauf auf den Katalysator 1 bis 1 000 Teile, je 1 Million Teile Katalysator, Cäsium, Rubidium oder eine Mischung davon aufgebracht wird.

Zur Herstellung von Ethylenoxid durch Oxidation von Ethylen mit Sauerstoff oder Luft werden Silberkatalysatoren eingesetzt, deren Herstellung seit langem bekannt und in verschiedenen Patentschriften beschrieben ist. Eine ganze Reihe großtechnischer Anlagen zur Herstellung von Ethylenoxid arbeiten nach dem Silberkatalysator-Verfahren. Dabei wird üblicherweise nur ein Bruchteil des eingesetzten Ethylens umgesetzt. Das umgesetzte Ethylen wird an dem mit Silber imprägnierten Trägermaterial mit Sauerstoff zum überwiegenden Teil in Ethylenoxid überführt, der Rest wird praktisch vollständig in Kohlendioxid und Wasser verwandelt.

Im Laufe der Zeit sind die verschiedensten Silberkatalysatoren entwickelt worden, und zwar mit dem Ziel, die Selektivität in bezug auf die bevorzugte Bildung von Ethylenoxid zu erhöhen und die Bildung von $CO_2$ und Wasser zurückzudrängen.

Bei steigenden Rohstoffpreisen und zunehmender Rohstoffverknappung kommt einer erhöhten Selektivität der Katalysatoren besondere wirtschaftliche Bedeutung zu. In den letzten Jahren sind in der Literatur im Prinzip zwei Wege beschrieben worden, mit denen Silber-Trägerkatalysatoren mit erhöhter Selektivität erhalten werden können. Der eine Weg beruht auf der Entwicklung neuer Silber-Trägerkatalysatoren, die sich von den älteren insbesondere durch eine spezielle Morphologie des aufgebrachten Silbers, durch ein spezielles Trägermaterial oder durch ausgewählte Promotoren unterscheiden.

Beispielsweise ist in der deutschen Offenlegungsschrift 23 00 512 ein Silber-Trägerkatalysator beschrieben, der dadurch erhalten wird, daß man auf Aluminiumoxid gleichzeitig mit dem Silber 0,000 4 bis 0,002 7 g-Äquivalente einer Kalium-, Rubidium- oder Cäsiumverbindung pro kg Katalysator aus wäßriger Lösung aufbringt.

Der andere Weg zur Herstellung von Silber-Trägerkatalysatoren mit erhöhter Selektivität beruht darauf, daß bei einem an sich fertigen Katalysator durch eine Nachbehandlung die Selektivität wesentlich verbessert wird. Dabei wird von einem Silber-Trägerkatalysator ausgegangen, der bereits zur Produktion von Ethylenoxid mehr oder weniger lang benützt worden ist.

Solche Verfahren zur Selektivitätsverbesserung sind in den deutschen Patentschriften 25 19 599, 26 11 856 und 26 36 680, in den deutschen Auslegeschriften 26 49 359 und 27 40 480 und in den deutschen Offenlegungsschriften 27 12 785, 27 46 976 und 27 51 767 beschrieben.

Bei dem Verfahren der deutschen Auslegeschrift 27 40 480 wird die Wirksamkeit von Silber-Trägerkatalysatoren dadurch verbessert, daß ein für die Direktoxidation bereits gebrauchter Katalysator mit einer inerten Flüssigkeit gewaschen wird und hierauf auf den Katalysator 1 bis 1 000 Teile, je 1 Million Teile Katalysator, Cäsium, Rubidium oder eine Mischung davon aufgebracht wird.

Nach der deutschen Offenlegungsschrift 27 51 767 wird eine Erhöhung der Selektivität bei Silber-Trägerkatalysatoren dadurch erreicht, daß man die Alkalimetalle Kalium, Rubidium and Cäsium mit einer Lösung aufbringt, die eine Verbindung von Kalium, Rubidium und/oder Cäsium, ein polares organisches Lösungsmittel, vorzugsweise einen Alkohol mit 1 bis 4 Kohlenstoffatomen, gegebenenfalls eine geringe Menge Wasser und ein Amin und/oder Ammoniak enthält. Als Amine werden Mono-, Di- oder Trialkylamine, Alkanolamine, mehrwertige Amine wie die Alkylendiamine, und Piperazine genannt. Sie werden in einer Menge von bis zu 30 Vol.-%, vorzugsweise von 0,1 bis 10 Vol.-%, bezogen auf das Gewicht der Lösung, eingesetzt. Die Lösung soll, wenn überhaupt, nur eine geringe Menge Wasser enthalten. Ausweislich aller Beispiele liegt die Wassermenge in den beschriebenen Lösungen unter 2 Gew.-%. In der deutschen Offenlegungsschrift 27 51 767 wird auch darauf hingewiesen, daß bei der bekannten Nachbehandlung von gebrauchten oder stabilisierten Silberkatalysatoren Lösungen von Salzen der schweren Alkalimetalle in einem polaren organischen Lösungsmittel eingesetzt werden, wobei das Lösungsmittel zur Verbesserung der Löslichkeit der Alkalisalze bis zu etwa 10 % Wasser enthalten kann.

Die Aufgabe der Erfindung liegt nun darin, das eingangs genannte Verfahren dahingehend weiter zu verbessern, daß ein noch wirksamerer Silber-Trägerkatalysator erhalten wird.

Dies wird erfindungsgemäß dadurch erreicht, daß zum Waschen eine Flüssigkeit verwendet wird, die aus 1 bis 20 Gew.-% eines Amins aus der Gruppe Propylamin, Butylamin, sekundäres Butylamin, Monoethanolamin und Ethylendiamin, 10 bis 50 Gew.-% Wasser und 30 bis 89 Gew.-% eines aliphatischen Alkohols mit 1 bis 3 C-Atomen oder eines aliphatischen Ketons mit 3 bis 6 C-Atomen, mit gegebenenfalls bis zu 10 Gew.-% Ammoniak, oder aus einer wäßrigen alkoholischen oder wäßrigen ketonischen Ammoniaklösung mit einer Ammoniakkonzentration von 1 bis 10 Gew.-%, einer Wasserkonzentration von 10 bis 50 Gew.-% und einer $C_1$- bis $C_3$-Alkohol- oder $C_3$- bis $C_6$-Ketonkonzentration von 40 bis 89 Gew.-% besteht, Gewichtsprozente jeweils bezogen auf das Gewicht der Flüssigkeit.

Bezüglich des Waschens des gebrauchten Silber-Trägerkatalysators gilt auch beim erfindungsgemäßen Verfahren das, was in der deutschen Offenlegungsschrift 27 40 480 ausgesagt ist. So kann das Waschen des gebrauchten Katalysators mit der erfindungsgemäß zu verwendenden Lösung durch

2

verschiedene Arbeitsweisen vorgenommen werden. Es kommt dabei lediglich darauf an, daß der Katalysator mit der Waschflüssigkeit in Berührung gebracht und anschließend davon abgetrennt wird. Das Waschen des Katalysators mit der Waschflüssigkeit kann auch mehrmals wiederholt werden. Dabei kann die prozentuale Zusammensetzung in bezug auf Amin, NH₃ und Lösungsmittel variiert werden. Die Zeit, innerhalb welcher der Katalysator mit der Waschflüssigkeit in Berührung ist, ist nicht kritisch ; sie kann von wenigen Minuten bis zu mehreren Stunden oder Tagen reichen und liegt in der Regel bei 5 Minuten bis 5 Tagen, vorzugsweise bei 0,5 bis 10 Stunden, insbesondere bei 1 bis 3 Stunden. Das Waschen kann beispielsweise dadurch erfolgen, daß der Katalysator in einem Behälter mit der Waschflüssigkeit überschüttet und anschließend von der Waschflüssigkeit durch Filtrieren, Abnutschen oder Abdekantieren abgetrennt wird. Es ist zweckmäßig, den Waschvorgang mehrere Male, vorzugsweise 2- bis 3mal, zu wiederholen, wobei jeweils eine frische (neue) Waschflüssigkeit verwendet wird. Neben der geschilderten Arbeitsweise kann das Waschen auch dadurch vorgenommen werden, daß der beispielsweise in einem oder mehreren Rohren als Festbett angeordnete Katalysator mit der Waschflüssigkeit übergossen wird, wobei die Waschflüssigkeit an einem Ende der Rohre aufgegeben und am anderen Ende der Rohre ablaufen gelassen wird. Auch dieser Waschvorgang kann mehrere Male wiederholt werden und wird vorzugsweise 1- bis 5mal durchgeführt, wobei zweckmäßigerweise jeweils eine frische (noch nicht gebrauchte) Waschflüssigkeit eingesetzt wird. Es kann von Vorteil sein, beim Übergießen des Katalysators (ein oder mehrere Male) die Waschflüssigkeit nicht gleich ablaufen zu lassen, sondern für einige Zeit, vorzugsweise für 0,5 bis 48 Stunden, im Rohr oder in den Rohren zu belassen, worauf dann der Abfluß freigegeben wird.

Es ist nicht erforderlich, den gesamten gebrauchten Katalysator zu waschen. Es kann der im Sinne der Erfindung angestrebte Effekt auch schon erreicht werden, wenn nur ein Teil, beispielsweise 30 bis 50 % des gebrauchten (gesamt vorliegenden) Katalysators, gewaschen wird.

Die Menge an Waschflüssigkeit richtet sich nach der zu waschenden Katalysatormenge und ist natürlich so zu bemessen, daß der Katalysator mit der Flüssigkeit in Berührung kommt. Zweckmäßigerweise beträgt die Menge an Waschflüssigkeit (pro Waschvorgang) in Gew.-Teilen oder Vol.-Teilen mindestens etwa 1/3 der Menge an Katalysator in Vol.-%, der zu waschen ist ; vorzugsweise werden etwa gleiche bis 5fache, insbesondere 2- bis 3fache Mengen an Waschflüssigkeit eingesetzt.

Im Falle des mehrmaligen Waschens hat es sich als vorteilhaft erwiesen, die Konzentration an Ammoniak und Amin fortlaufend zu erniedrigen und die Alkohol- und Ketonmenge zu erhöhen. Dadurch wird erreicht, daß neben dem Ammoniak bzw. Amin auch ein Großteil des Wassers vom Katalysator entfernt und seine Trocknung erleichtert wird.

Die Temperatur des Katalysators und der Waschflüssigkeit während des Waschens ist nicht kritisch und ist mehr von praktischen Gesichtspunkten bestimmt. Sie liegt zweckmäßigerweise unter dem Siedepunkt der verwendeten inerten Flüssigkeit und des eingesetzten Amins. Beim Einsatz der Ammoniak enthaltenden Flüssigkeiten sind Temperaturen von 15 bis 30 °C zweckmäßig. Ein evtl. Verdampfen von Waschflüssigkeit, beispielsweise des Alkohols, oder ein Ausgasen des Ammoniaks oder des Amins kann durch Anwendung von Druck verhindert werden. Dabei wird der Druck zweckmäßigerweise bei 1 bis 20 bar, vorzugsweise bei 1 bis 5 bar liegen.

Nach dem Waschen erfolgt erfindungsgemäß das Aufbringen von 1 bis 1 000 mg pro kg Katalysator an Cäsium, Rubidium oder einer Mischung dieser beiden (wobei das Mengenverhältnis beliebig sein kann). Auch beim erfindungsgemäßen Verfahren gilt diesbezüglich im wesentlichen das in der deutschen Auslegeschrift 27 40 480, ab Spalte 5, Ausgesagte. Das Aufbringen von Cäsium und/oder Rubidium auf den gewaschenen Katalysator, der zweite Verfahrensschritt des erfindungsgemäßen Verfahrens, kann direkt nach dem ersten Verfahrensschritt, dem Waschen, vorgenommen werden oder auch erst nachdem der Katalysator einer Trocknung unterworfen worden ist, wobei vom Waschen nach Abtrennen der Waschflüssigkeit gegebenenfalls verbliebene Flüssigkeitsreste entfernt werden. Die Trocknung kann beispielsweise durch Hindurchleiten eines Inertgases und/oder Erwärmen des Katalysators erreicht werden, wobei zur Beschleunigung des Trocknungsvorganges auch Unterdruck angewendet werden kann. Die bei der Erwärmung angewendete Temperatur ist nicht kritisch, sie richtet sich nach der eingesetzten Waschflüssigkeit und wird, sofern bei Atmosphärendruck getrocknet wird, etwa dem Siedepunkt der Waschflüssigkeit entsprechen. Zweckmäßige Trocknungstemperaturen sind beispielsweise etwa 20 bis 250 °C, vorzugsweise 50 bis 150 °C. Wenn man mit Unterdruck trocknet kann auch schon bei Zimmertemperatur, das heißt bei 15 bis 25 °C oder beispielsweise bei 25 bis 30 °C eine vollständige Verdampfung der am Katalysator verbliebenen Waschflüssigkeit, nachdem der größte Teil bereits abgetrennt wurde, erreicht werden. Erwärmen und gleichzeitiges Überleiten eines Inertgases beschleunigt das Trocknen ebenfalls. Als Inertgas werden zweckmäßigerweise unbrennbare, die Verbrennung nicht fördernde Gase wie Stickstoff oder Kohlendioxid verwendet. Sofern Zündquellen ausgeschatet werden und/oder ein großer Überschuß des Gases verwendet wird, der mit den flüchtigen Stoffen keine zündfähigen Gemische bildet, können auch andere Gase, insbesondere Luft, verwendet werden.

Für das Aufbringen von Cäsium und/oder Rubidium auf den gewaschenen (getrockneten oder nicht getrockneten) Katalysator können verschiedene arbeitsweisen gewählt werden ; wichtig ist lediglich, daß die angegebenen Mengen von Cäsium, Rubidium oder auch Abmischungen davon auf den Katalysator (und zwar auf den gesamten gebrauchten Katalysator, also auch auf gegebenenfalls nicht gewaschene Teilmengen) aufgebracht werden.

Zweckmäßigerweise erfolgt das Aufbringen von Cäsium und/oder rubidium durch Benetzen (Tränken, Imprägnieren) des Katalysators mit einer Imprägnierflüssigkeit, die eine oder mehrere Verbindungen von Cäsium und/oder Rubidium enthält.

Die Imprägnierflüssigkeit soll die Cäsium und/oder Rubidiumverbindungen in möglichst fein verteilter Form enthalten. Die genannten Verbindungen können in Dispersion oder Emulsion vorliegen, vorzugsweise werden sie jedoch in gelöster Form angewendet (Imprägnierlösung). Bevorzugte Lösungsmittel sind aliphatische, alicyclische oder aromatische Ketone, vorzugsweise aliphatische Ketone mit 3 bis 10 C-Atomen, wie Aceton, Methylethylketon, Ethylpropylketon ; und aliphatische, alicyclische oder aromatische Alkohole, vorzugsweise aliphatische oder alicyclische Alkohole mit 1 bis 6 C-Atomen.

Besonders bevorzugt werden aliphatische (geradkettige oder verzweigte) Alkohole mit 1 bis 6 C-Atomen, insbesondere mit 1 bis 3 C-Atomen, wie Methanol, Ethanol, Propanol, Isopropanol.

Die organischen Lösungsmittel können für sich allein (wobei auch Mischungen untereinander möglich sind) eingesetzt werden oder in Mischung mit Wasser. Auch reines Wasser ist als Lösungsmittel verwendbar. Sofern Mischungen aus organischen Lösungsmitteln und Wasser eingesetzt werden, sind solche bevorzugt, deren Wassergehalt bis zu 40 Gew.-%, vorzugsweise bis zu 20 Gew.-%, bezogen auf Gesamtflüssigkeit, ausmacht.

Die Art der Cäsium- und Rubidiumverbindungen ist für den erfindungsgemäßen Effekt nicht entscheidend. In der Regel werden die gewählte Verbindung oder die gewählten Verbindungen Cäsium und Rubidium in Gestalt der entsprechenden Kationen enthalten. In Verbindung mit welchem Rest (Anion) Cäsium und Rubidium vorliegt, ist von geringer Bedeutung. Es kann sich um anorganische oder organische Reste handeln. Allerdings sollte dieser Rest nicht aus Stoffen bestehen, die insbesondere nach Behandlung mit der gasförmigen Reaktionsmischung zur Herstellung von Ethylenoxid als sogenanntes Katalysatorengift wirken. Geeignete Cäsium- und Rubidiumverbindungen sind :

anorganische Verbindungen, vorzugsweise anorganische Salze, beispielsweise Sulfate, Nitrite, Nitrate, Silikate, Carbonate, Bicarbonate ; Hydroxide und Oxide ; organische Verbindungen, vorzugsweise organische Salze, beispielsweise Formiate, Acetate, Oxalate, Malonate, Succinate, Butyrate, Laurate, Stearate, Lactate, Tartrate und Benzoate ; und Alkoholate beispielsweise Methylat, Ethylat und Phenolat. Bevorzugt eingesetzt werden anorganische oder organische Salze, insbesondere die Formiate, Acetate, Carbonate, Bicarbonate, Nitrate ; die Hydroxide ; und die Alkoholate von aliphatischen Alkoholen, vorzugsweise mit 1 bis 6, besonders mit 1 bis 3 C-Atomen. Es können sowohl eine als auch mehrere Cäsium- oder Rubidiumverbindungen eingesetzt werden, Mischungen von Cäsium- und Rubidiumverbindungen sind ebenfalls geeignet.

Die Konzentration der Cäsium- und/oder Rubidiumverbindungen in der Imprägnierflüssigkeit ist nicht kritisch ; sie wird sich im allgemeinen nach der Löslichkeit der Verbindungen richten. Entscheidend ist lediglich, daß nach dem Behandeln des Katalysators mit der Imprägnierflüssigkeit eine Konzentration von 1 bis 1 000 mg/kg an Cäsium und/oder Rubidium auf dem Katalysator eingestellt ist (die Konzentrationsangabe für Cäsium oder Rubidium auf dem Katalysator bezieht sich nur auf das Cäsium- oder Rubidiummetall, der Rest in der gewählten Verbindung bleibt bei dieser Angabe außer Betracht). Es empfiehlt sich, in der Imprägnierflüssigkeit (Imprägnierlösung) eine Mindestkonzentration von 0,000 3 Gew.-% an Cäsium und/oder Rubidium zu nehmen. Als besonders zweckmäßig hat sich eine Konzentration von 0,003 bis 1,0 Gew.-%, vorzugsweise von 0,005 bis 0,5 Gew.-% an Cäsium- und/oder Rubidiumverbindung, bezogen auf Imprägnierlösung, erwiesen. Jedoch richtet man sich bei der Einstellung der Konzentration der Imprägnierlösung stets nach der erwünschten Konzentration an Rubidium oder Cäsium auf dem Katalysator.

Auch die Menge an Imprägnierflüssigkeit kann in weiten Grenzen variiert werden. Man wird sich dabei nach der Menge an zu behandelndem Katalysator richten, so daß alle Katalysatorteilchen auch vollständig benetzt werden. Nach oben ist somit die Menge der Imprägnierflüssigkeit von ihrer Wirkung her nicht begrenzt. Im allgemeinen wird man 75 bis 150 Vol.-% Imprägnierflüssigkeit, bezogen auf zu behandelnden Katalysator, nehmen.

Die Behandlung des gewaschenen Katalysators mit der Imprägnierflüssigkeit, um 1 bis 1 000 mg/kg, vorzugsweise 3 bis 300 mg/kg, Cäsium und/oder Rubidium auf den Katalysator aufzubringen, kann nach verschiedenen Arbeitsweisen durchgeführt werden. Geeignete Verfahren sind in der deutschen Auslegeschrift 27 40 480 beschrieben. Eine zweckmäßige Arbeitsweise ist die Tränkung (Benetzung, Imprägnierung), wobei der Katalysator mit der Imprägnierflüssigkeit übergossen und der überschüssige Teil vom Katalysator abgetrennt wird (durch Abdekantieren oder einfaches Abfließenlassen). Dies wird beispielsweise in einem Behälter, gegebenenfalls unter Rühren, oder an dem in einem oder mehreren Rohren als Festbett angeordneten Katalysator ausgeführt. Diese letzte Methode empfiehlt sich besonders bei Großanlagen, in denen der Katalysator bereits in den Rohren des Reaktors vorliegt. Das Übergießen (Fluten) kann einmal oder mehrmals (mit der abgetrennten oder mit einer neu bereiteten Imprägnierflüssigkeit) durchgeführt werden.

Das Aufbringen von 1 bis 1 000 Teilen, vorzugsweise 3 bis 300 Teilen Cäsium und/oder Rubidium, je 1 Million Teile Katalysator, auf den gewaschenen Katalysator kann auch in zwei oder mehr, vorzugsweise zwei bis fünf (zeitlich verschobenen) Schritten erfolgen, wobei der Katalysator nach jedem Behandlungsschritt wieder für die Direktoxidation von Ethylen zu Ethylenoxid mit molekularem Sauerstoff oder Luft eingesetzt wird. Die zeitliche Verschiebung zwischen den einzelnen Behandlungsschritten, d. h. die Zeitspanne während der der Katalysator nach einer Behandlung wieder zur Direktoxidation eingesetzt worden ist, kann innerhalb weiter Grenzen variieren ; sie liegt zweckmäßigerweise bei einer Woche bis zu mehreren Monaten, vorzugsweise bei 1 bis 20 Wochen, insbesondere bei 3 bis 5 Wochen. Nach oben ist die Grenze der Zeitspanne mehr nach technisch-wirtschaftlichen Gesichtspunkten begrenzt und die untere Grenze liegt zweckmäßigerweise nicht unter einer Stunde.

Die Menge an Cäsium und/oder Rubidium, die pro Behandlungsschritt auf den gewaschenen Katalysator aufgebracht wird, kann innerhalb des Bereiches 1 bis 1 000 mg/kg, vorzugsweise 3 bis 300 mg/kg, variiert werden. In der Regel wird man beim ersten Behandlungsschritt mindestens 1 mg/kg, vorzugsweise 3 bis 100 mg/kg auf den Katalysator aufbringen und bei den folgenden Behandlungsschritten jeweils die gleiche oder eine Teilmenge davon.

Nach dem Behandeln des gewaschenen Katalysators mit Imprägnierflüssigkeit (nach einer der vorhin beschriebenen Methoden), ist es zweckmäßig, wenn die nach Abtrennen des größten Teils der Imprägnierflüssigkeit am Katalysator gegebenenfalls verbliebenen Flüssigkeitsreste entfernt werden, was nach einem der oben beschriebenen Trocknungsverfahren erfolgen kann. Die dabei gegebenenfalls angewendeten Temperaturen richten sich, ähnlich wie dort, nach der zu entfernenden (verdampfenden) Imprägnierflüssigkeit und liegen bei 50 bis 250 °C, vorzugsweise bei 50 bis 150 °C, insbesondere bei 70 bis 120 °C.

Das erfindungsgemäße Verfahren ist unabhängig von der Art (beispielsweise Zusammensetzung, Aufbau, Struktur) des Silberkatalysators selbst. Jeder für die Direktoxidation von Ethylen zu Ethylenoxid mit molekularem Sauerstoff oder Luft geeignete Silberkatalysator kommt für das erfindungsgemäße Verfahren in Betracht. Silberkatalysatoren für die Direktoxidation von Ethylen zu Ethylenoxid mit molekularem Sauerstoff oder Luft sind, ebenso wie die Direktoxidation selbst, in der Literatur eingehend beschrieben, beispielsweise in den folgenden US-Patentschriften : 2 615 899, 3 899 445 und 3 962 136.

Die in Rede stehenden Silberkatalysatoren bestehen im allgemeinen aus 1 bis 40 Gew.-% (bezogen auf den gesamten Katalysator) an Silber auf einem Trägermaterial und gegebenenfalls aus mehr oder weniger großen Mengen verschiedenster Promotoren oder Koaktivatoren. Dabei ist das Silber als Metall auf den inneren und äußeren Oberflächen des vorzugsweise porösen Trägermaterials niedergeschlagen und möglichst gleichmäßig über dessen gesamte Oberfläche verteilt. Die Morphologie des auf dem Trägermaterial aufgebrachten Silbers kann innerhalb weiter Grenzen variieren. Im allgemeinen hat es die Gestalt von kugelförmigen Teilchen mit einem Durchmesser von 0,01 bis 10 µm. Das Trägermaterial besteht zweckmäßigerweise aus porösen, hitzebeständigen und unter den bei der Direktoxidation von Ethylen auftretenden Bedingungen inert bleibenden Materialien. Beispiele für solche Materialien sind Aluminiumverbindungen, vorzugsweise Aluminiumoxide verschiedenster Struktur, Magnesiumoxide, Kieselgur, Bimsstein, Siliciumdioxid, Siliciumcarbid, Tone, Korund, Zeolithe, Metalloxide und ähnliche. Besonders bevorzugte Trägermaterialien sind α-Aluminiumoxide, da sie einen weitgehend gleichmäßigen Porendurchmesser aufweisen. Sie werden charakterisiert insbesondere durch die spezifische Oberfläche (m²/g), das spezifische Porenvolumen (cm³/g) und den mittleren Porendurchmesser (µm). Die Trägermaterialien werden in der Regel in Form von Granulaten, Kugeln, Stückchen, Ringen oder dergleichen eingesetzt.

Das erfindungsgemäße Zweistufen-Verfahren bezieht sich auf gebrauchte Silberkatalysatoren. Der Ausdruck « gebraucht » bedeutet in diesem Zusammenhang, daß der Katalysator zur Umsetzung von Ethylen zu Ethylenoxid mit molekularem Sauerstoff oder Luft bereits eingesetzt war, wobei es belanglos ist, ob dabei seine ursprüngliche Selektivität nachgelassen hat oder nicht. Die Zeit, während der der Katalysator für die Oxidation von Ethylen zu Ethylenoxid vor der erfindungsgemäßen Behandlung in Gebrauch war, kann innerhalb weiter Grenzen variieren ; sie kann von wenigen Wochen (1 bis 3) bis mehrere Jahre (1 bis 10) und darüber schwanken. Dabei kann der Katalysator in seiner Wirksamkeit nachgelassen, d. h. in der Selektivität abgenommen haben (was in der Regel nach längerer Gebrauchszeit eintritt), er kann auch die ursprüngliche Selektivität beibehalten haben.

Mit dem erfindungsgemäßen Waschen und Aufbringen von Cäsium und/oder Rubidium gelingt es also, Silber-Trägerkatalysatoren, die bereits für die Direktoxidation von Ethylen mit molekularem Sauerstoff oder Luft in Gebrauch waren, in ihrer Wirksamkeit erheblich zu verbessern. Die Wirksamkeit eines Katalysators kann ausgedrückt werden als Umsatz (in %) von Ethylen bei gegebener Temperatur oder als molares Verhältnis von in Ethylenoxid umgesetztes Ethylen, das ist seine Selektivität. Ein Katalysator ist umso wirksamer, je mehr Ethylen bei einer bestimmten Temperatur umgesetzt wird, je höher die Selektivität bei einem bestimmten Umsatz ist und je niedriger die Temperatur zur Erzielung eines bestimmten Umsatzes ist.

Mit dem erfindungsgemäßen Verfahren wird nicht nur die Selektivität von gebrauchten Silber-Trägerkatalysatoren, sondern auch der Umsatz wesentlich gesteigert. Darüberhinaus ist es auch möglich, bei erfindungsgemäß behandelten Katalysatoren die Reaktionstemperatur abzusenken — bei gleichem oder sogar erhöhtem Umsatz. Dies ist deshalb besonders bedeutungsvoll, weil mit niedrigerer Reaktionstemperatur die Bildung unerwünschter Nebenprodukte wie Kohlendioxid, Formaldehyd, Acetaldehyd, stark zurückgeht. Angesichts der großen Mengen Ethylenoxid, die nach dem Ethylen-Oxidationsverfahren produziert werden, kommt einer Ausbeutesteigerung auch nur um wenige Prozent oder sogar um Zehntelprozent erhebliche wirtschaftliche Bedeutung zu. Was das erfindungsgemäße Verfahren ferner auszeichnet, ist der Umstand, daß es in den üblichen Großproduktions-Anlagen (mit den handelsüblichen Silber-Trägerkatalysatoren) ohne nennenswerten zusätzlichen Aufwand an Energie, Investitionen und Material durchgeführt werden kann.

Die Erfindung wird nun an Beispielen näher erläutert.

Die folgenden Beispiele und Vergleichsbeispiele werden in einem Versuchsreaktor aus einem senkrecht stehenden Reaktionsrohr aus Chromvanadiumstahl mit einer lichten Weite von 30 mm und einer Länge von 300 mm durchgeführt. Das mit einem Mantel versehene Reaktionsrohr wird mit heißem Öl, das durch den Mantel strömt, beheizt. Das Reaktionsrohr ist bis zu einer Höhe von 200 mm mit $\alpha$-$Al_2O_3$-Pellets gefüllt ; diese Füllung dient zur Vorheizung des Einsatzgases. Auf der inerten Füllung liegt der zu prüfende Katalysator. Das Einsatzgas tritt unten (drucklos) in das Reaktionsrohr ein und verläßt es am Kopf.

Das eingesetzte Gasgemisch besteht aus

| | | |
|---|---|---|
| $C_2H_4$ | 28 | Vol.-% |
| $CH_4$ | 53 | Vol.-% |
| $O_2$ | 8 | Vol.-% Gasgemisch I |
| $CO_2$ | 5 | Vol.-% |
| $N_2$ | 6 | Vol.-% |
| Vinylchlorid | 0,000 2 | Vol.-% (Inhibitor) |

oder aus

| | |
|---|---|
| $C_2H_4$ | 4 Vol.-% |
| $O_2$ | 5 Vol.-% Gasgemisch II |
| $CO_2$ | 4 Vol.-% |
| $N_2$ | 87 Vol.-% |

Die Raum-Zeit-Geschwindigkeit beträgt :

$$250 \cdot \frac{\text{Raumteile Gas}}{\text{Stunde} \cdot \text{Raumteile Katalysator}}$$

Das am Reaktorausgang austretende Gas wird gaschromatographisch analysiert und Umsatz und Selektivität berechnet. Die Temperatur des Wärmeträgermediums wird so lange variiert, bis ein Konstanter Ethylenumsatz von 7 % bei Gasgemisch I, bzw. von 35 % bei Gasgemisch II erzielt wird.

Die Laufzeit der Versuche wird so gewählt, daß am Ende keine Veränderung der Meßwerte mehr erfolgt. Das ist normalerweise bei einer Laufzeit von 200 Stunden der Fall.

Für die Versuche werden handelsübliche Silber-Trägerkatalysatoren eingesetzt. Sie bestehen aus 10 % Silber (Teilchendurchmesser 1 bis 5 μm) auf $\alpha$-$Al_2O_3$ als Trägermaterial, das die Form von Ringkörpern mit einer Länge von 8 mm, einem äußeren Durchmesser von 8 mm und einem inneren Durchmesser von 2 mm — Katalysator I — oder das die Form von Zylindern mit einem Durchmesser und einer Höhe von etwa 5 mm — Katalysator II — besitzt ; die spezifische Oberfläche liegt bei 0,1 bis 0,5 m²/g.

Der auf dem Katalysator aufgebrachte Cäsium- und/oder Rubidiumgehalt wird durch Atomabsorptionsspektroskopie bestimmt (siehe Monographie : « Atomabsorptionsspektroskopie », Bernhard Wells Verlag Chemie, 1972. Seite 114 ff.). Die Bestimmung wird in einer Luft-Acetylenflamme durchgeführt und die Cs- oder Rb-Extinktion in Emission gemessen.

Beispiel 1

50 g des oben beschriebenen handelsüblichen Katalysators II, der 4 Jahre zur Herstellung von Ethylenoxid durch Direktoxidation von Ethylen mit Sauerstoff eingesetzt war, werden in einem 200 ml-Erlenmeyerkolben mit einer Lösung, bestehend aus 25 ml Methanol, 10 ml destilliertem Wasser und 15 ml einer 25 %igen wäßrigen Ammoniaklösung bei 20 °C überschüttet und 10 Stunden lang stehengelassen. Nach dem Abdekantieren der Waschflüssigkeit wird der Katalysator zweimal mit Methanol, das 10 Gew.-% Wasser enthält, je 0,5 Stunden stehengelassen und das Methanol abdekantiert. Sodann wird der Katalysator 12 Stunden bei 120 °C im Trockenschrank getrocknet. Der auf 20 °C abgekühlte Katalysator wird nun mit 100 ml einer Lösung aus 50 ml Methanol, 1 g destilliertem Wasser und 0,02 g Cäsiumnitrat übergossen und 1 Stunde bei Raumtemperatur stehengelassen. Nach dem Abdekantieren der Tränklösung wird der Katalysator 3 Stunden bei 120 °C im Trockenschrank getrocknet. Die Cäsium-konzentration auf dem Katalysator beträgt 85 mg/kg.

Der so behandelte Katalysator wird nun in den Versuchsreaktor eingefüllt und in der beschriebenen Weise mit Gasgemisch I getestet : Die Selektivität ist von vorher 68 % bei 7 % Ethylenumsatz und einer Temperatur von 260 °C auf 75 % bei gleichem Ethylenumsatz, aber bei 227 °C, gestiegen.

## Vergleichsbeispiel 1

Es wird wie in Beispiel 1 vorgegangen, wobei jedoch die Ammoniakwäsche weggelassen wird.

Es ergibt sich eine Steigerung der Selektivität von 68 % bei 260 °C auf 71 % bei 250 °C, bei jeweils konstantem Umsatz von 7 %.

## Beispiel 2

50 g des in Beispiel 1 benutzten Katalysators II werden in einem 200 ml Erlenmeyerkolben mit einer Lösung aus 40 ml Ethanol (Butanon vergällt), 5 ml Wasser und 5 ml Ethylendiamin 10 Stunden lang bei 40 °C stehengelassen. Nach dem Abdekantieren der Waschflüssigkeit wird der Katalysator dreimal mit je 100 ml Ethanol gewaschen, wobei vor dem Abdekantieren jeweils 0,5 Stunden lang stehengelassen wird. Anschließend wird der gewaschene Katalysator 24 Stunden lang bei 170 °C getrocknet. Der auf 30 °C abgekühlte Katalysator wird nun 1 Stunde lang mit einer Cäsiumlösung, wie in Beispiel 1 beschrieben, getränkt.

Beim Test mit Gasgemisch II steigt die Selektivität von 65 % bei 252 °C und 35 % Ethylenumsatz auf 73 % bei 237 °C.

## Vergleichsbeispiel 2

Es wird wie in Beispiel 2 vorgegangen, wobei jedoch das Ethylendiamin weggelassen wird. Die Selektivität steigt von 65 % bei 252 °C auf 68 % bei 245 °C.

## Beispiel 3

50 g des Katalysators I, der 7 Jahre lang zur Herstellung von Ethylenoxid durch Direktoxidation von Ethylen mit Sauerstoff in Verwendung war, werden in einem 200 ml Erlenmeyerkolben mit einer Lösung aus 25 ml Methanol, 15 ml Wasser und 20 ml von 25 %iger wäßriger Ammoniaklösung bei 25 °C übergossen und 5 Stunden lang leicht gerührt. Nach dem Abfiltrieren der Waschflüssigkeit wird der Katalysator zweimal mit je 50 ml Methanol, das 10 Gew.-% Wasser enthält, jeweils 2 Stunden lang gerührt, worauf das Methanol abgegossen wird. Der noch feuchte Katalysator wird nun mit 50 ml einer Tränklösung aus 1 000 mg Cäsiumnitrat und 1 kg Methanol 5 Stunden lang bei 25 °C stehengelassen. Hierauf wird die Tränklösung abgegossen und der Katalysator 24 Stunden lang bei 150 °C im Trockenschrank getrocknet. Die Cäsiumkonzentration auf dem Katalysator beträgt 130 mg/kg.

Beim Test mit Gasgemisch I steigt die Selektivität von vorher 66 % bei 7 % Ethylenumsatz und 240 °C auf 76 % bei demselben Ethylenumsatz, aber 225 °C.

## Vergleichsbeispiel 3

Es wird wie in Beispiel 3 vorgegangen, wobei jedoch in der Waschflüssigkeit der Ammoniak fehlt. Die Selektivität steigt von 66 % bei 240 °C auf 75 % bei 225 °C.

## Beispiele 4 bis 7

Diese Beispiele werden analog dem Beispiel 1 durchgeführt, mit den in der folgenden Tabelle angegebenen Waschlösungen und Vorgangsweisen.

(Siehe die Tabelle, Seite 8)

Tabelle

| Beispiel Nr. | Katalysator Typ | g | Gasgemisch | 100 g Lösungsmittel aus Amin, Ammoniak Gew.-% | | $H_2O$ Gew.-% | Alkohol, Keton Gew.-% | | Einwirkdauer bei 20-30 °C h | Cs-Tränklösung | Reaktionstemperatur °C | Selektivität bei 7 % $C_2H_4$-Umsatz % |
|---|---|---|---|---|---|---|---|---|---|---|---|---|
| 4 | II | 50 | I | sec. Butylamin | 15 | 30 | Aceton | 55 | 10 | 350 mg Cs/kg Aceton | 227 | 76,5 |
| 5 | II | 50 | I | Ethylendiamin<br>$NH_3$ 25 %ig | 10<br>10 | 30 | Methanol | 50 | 2 mai je 2 | 300 mg Cs/kg Methanol | 226 | 77,2 |
| 6 | II | 50 | I | Monoethanol-amin | 20 | 20 | Isopropanol | 60 | 3 mal je 3 | 200 mg Cs/kg Isopropanol | 228 | 76,3 |
| 7 | I | 50 | I | $NH_3$ 25 %ig<br>sec. Butylamin | 2<br>10 | 10 | Methanol<br>Aceton | 50<br>28 | 1 mal 24 | 300 mg Cs/kg Methanol | 227 | 76,8 |

0 025 963

**Anspruch**

Verfahren zur Verbesserung der Wirksamkeit von Silber-Trägerkatalysatoren für die Direktoxidation von Ethylen zu Ethylenoxid mit molekularem Sauerstoff oder Luft, wobei der für die Direktoxidation gebrauchte Katalysator mit einer inerten Flüssigkeit gewaschen wird und hierauf auf den Katalysator 1 bis 1 000 Teile, je 1 Million Teile Katalysator, Cäsium, Rubidium oder eine Mischung davon aufgebracht wird, dadurch gekennzeichnet, daß zum Waschen eine Flüssigkeit verwendet wird, die aus 1 bis 20 Gew.-% eines Amins aus der Gruppe Propylamin, Butylamin, sekundäres Butylamin, Monoethanolamin und Ethylendiamin, 10 bis 50 Gew.-% Wasser und 30 bis 89 Gew.-% eines aliphatischen Alkohols mit 1 bis 3 C-Atomen oder eines aliphatischen Ketons mit 3 bis 6 C Atomen, mit gegebenenfalls bis zu 10 Gew.-% Ammoniak, oder aus einer wäßrigen alkoholischen oder wäßrigen ketonischen Ammoniaklösung mit einer Ammoniakkonzentration von 1 bis 10 Gew.-%, einer Wasserkonzentration von 10 bis 50 Gew.-% und einer $C_1$- bis $C_3$-Alkohol- oder $C_3$- bis $C_6$-Ketonkonzentration von 40 bis 89 Gew.-% besteht, Gewichtsprozente jeweils bezogen auf das Gewicht der Flüssigkeit.

**Claim**

Process for improving the activity of silver-support catalysts for the direct oxidation of ethylene to ethylene oxide by means of molecular oxygen or air, whereby the catalyst used for the direct oxidation is washed with an inert liquid and on the catalyst is then deposited 1 to 1 000 parts, per 1 million parts of catalyst, of cesium, rubidium or a mixture thereof, characterised in that for washing a liquid is used which consists of 1 to 20 percent by weight of an amine selected from the group consisting of propylamine, butylamine, secondary butylamine, monoethanolamine and ethylenediamine, 10 to 50 percent by weight of water and 30 to 89 percent by weight of an aliphatic alcohol having 1 to 3 carbon atoms or an aliphatic ketone having 3 to 6 carbon atoms, which optionally contains ammonia in a quantity of up to 10 percent by weight, or of an aqueous alcoholic or an aqueous ketonic ammonia-solution having an ammonia concentration of 1 to 10 percent by weight, a water concentration of 10 to 50 percent by weight and a $C_1$- to $C_3$-alcohol- or $C_3$- to $C_6$-ketone-concentration of 40 to 89 percent by weight, the percentages by weight being in each case relative to the weight of the liquid.

**Revendication**

Procédé pour améliorer l'activité de catalyseurs à l'argent sur support pour l'oxydation directe de l'éthylène en oxyde d'éthylène à l'aide d'oxygène moléculaire ou d'air, selon lequel on lave avec un liquide inerte le catalyseur ayant servi à l'oxydation directe puis l'on applique ou dépose sur le catalyseur 1 à 1 000 parties, par million de parties du catalyseur, de césium, de rubidium ou d'un mélange de césium et de rubidium, procédé caractérisé en ce qu'on utilise pour le lavage un liquide qui consiste en 1 à 20 % en poids d'une amine choisie dans l'ensemble formé par la propylamine, la butylamine, la butylamine secondaire, la monoéthanolamine et l'éthylènediamine, 10 à 50 % en poids d'eau et 30 à 89 % en poids d'un alcool aliphatique ayant 1 à 3 atomes de carbone ou d'une cétone aliphatique ayant 3 à 6 atomes de carbone, avec éventuellement jusqu'à 10 % en poids d'ammoniac, ou consistant en une solution hydroalcoolique ou hydrocétonique d'ammoniac ayant une concentration en ammoniac de 1 à 10 % en poids, une concentration en eau de 10 à 50 % en poids et une concentration en alcool comportant 1 à 3 atomes de carbone ou en une cétone ayant 3 à 6 atomes de carbone de 40 à 89 % en poids, les pourcentages en poids se rapportant à chaque fois au poids du liquide.